# EUROPÄISCHE PATENTSCHRIFT

(11) **EP 1 311 655 B1**
(45) Veröffentlichungstag und Bekanntmachung des Hinweises auf die Patenterteilung: **07.06.2006**
(21) Anmeldenummer: 01962833.8
(22) Anmeldetag: 05.07.2001
(51) Int. Cl.: C12M 1/34

(54) **VORRICHTUNG UND VERFAHREN ZUM ELEKTRISCHEN KONTAKTIEREN VON IN EINER FLÜSSIGKEIT IN SUSPENSION BEFINDLICHEN BIOLOGISCHEN ZELLEN**
DEVICE AND METHOD FOR MAKING ELECTRICAL CONTACT WITH BIOLOGICAL CELLS SUSPENDED IN A FLUID
DISPOSITIF ET PROCEDE POUR METTRE EN CONTACT ELECTRIQUE DES CELLULES BIOLOGIQUES EN SUSPENSION DANS UN LIQUIDE

(30) Priorität: 05.07.2000 DE 10032568
(43) Veröffentlichungstag der Anmeldung: 21.05.2003
(73) Patentinhaber: NMI NATURWISSENSCHAFTLICHES UND MEDIZINISCHES INSTITUT AN DER UNIVERSITÄT TÜBINGEN, 72770 Reutlingen (DE)
(72) Erfinder: STETT, Alfred, 72770 Reutlingen (DE); HÄMMERLE, Hugo, 72072 Tübingen-Bühl (DE); NISCH, Wilfried, 72072 Tübingen (DE); KNOTT, Thomas, 72127 Wankheim (DE)
(74) Vertreter: Duhme, Torsten
(86) Internationale Anmeldenummer: PCT/EP2001/007713
(87) Internationale Veröffentlichungsnummer: WO 2002/003058

(56) Entgegenhaltungen:
- WO-A-00/34776
- DE-A- 19 712 309

## Beschreibung

Die vorliegende Erfindung betrifft eine Vorrichtung zum elektrischen Kontaktieren von in einer Flüssigkeit in Suspension befindlichen biologischen Zellen, mit einem zumindest eine Öffnung aufweisenden Substrat, mit Mitteln zum Immobilisieren einer Zelle auf der Öffnung, und mit mindestens einer Elektrode für die elektrische Kontaktierung einer immobilisierten Zelle, wobei die Elektrode elektrisch mit einem in einer Kontaktspitze mündenden Kontaktkanal in Verbindung steht, über den auf die Zelle ein erster hydrodynamischer Unterdruck ausübbar ist.

Die Erfindung betrifft ferner ein Verfahren zum elektrischen Kontaktieren von in einer Flüssigkeit in Suspension bef indlichen biologischen Zellen mit den Schritten:
- Immobilisieren einer Zelle oberhalb einer in einem Substrat vorgesehenen Öffnung, und
- Kontaktieren der immobilisierten Zelle über zumindest eine Elektrode, wobei, die Elektrode durch eine Kontaktspitze hindurch mit der Zelle elektrisch in Kontakt gelangt, und wobei durch einen in der Kontaktspitze mündenden Kontaktkanal auf eine Zellmembran der Zelle ein erster hydrodynamischer Unterdruck ausgeübt wird.

Eine derartige Vorrichtung und ein entsprechendes Verfahren sind aus der WO 00/34776 bekannt.

Aus DE 197 12 309 A1 ist eine Mikroelektrodenanordnung bekannt, bei der die Zellen in Mikroküvetten eingefangen werden, an deren Boden sich eine Elektrode befindet. Die Elektrode ist mit einem zentralen Ansaugkanal versehen, in dem über geeignete Verbindungskanäle, die unterhalb der Elektroden verlaufen, ein Unterdruck erzeugt werden kann. Auf diese Weise ist es möglich, einzelne Zellen zielgerichtet an die Elektroden heranzuziehen und sie unter einem gewissen Kontaktdruck auf den Elektroden zu immobilisieren. Mit der bekannten Vorrichtung können dann Messungen an den Zellen, jedoch nur von deren Membranaußenseite her, durchgeführt werden.

Derartige Mikroelektrodenanordnungen werden allgemein zum Untersuchen von biologischen Zellen verwendet. Die Mikroelektrodenanordnungen dienen dabei z.B. zum Stimulieren der Zellen oder zum Ableiten von Potentialen. Die Untersuchungen können in einer biologischen Umgebung oder in einer artifiziellen Umgebung durchgeführt werden. Die Anordnungen umfassen dabei in einem Trägerkörper, also auf einem Substrat, eine Vielzahl von Mikroelektroden, deren Abmessungen etwa in der Größenordnung der Zellen liegen, also im Bereich von einigen µm bis einigen 10 *µ*m.

Bei herkömmlichen Mikroelektrodenanordnungen ist man mehr oder weniger auf den Zufall angewiesen, ob sich die eine oder die andere Zelle auf einer bestimmte Elektrode niederläßt oder nicht. In der Praxis lassen sich die Zellen im allgemeinen nur unter teilweiser Überdeckung auf einer Elektrode nieder, so daß die Stimulation der Zelle bzw. die Ableitung eines Zellpotentials auf diese Teilfläche beschränkt ist.

Diese Nachteile werden durch die aus DE 197 12 309 A1 bekannte Vorrichtung zwar größtenteils beseitigt, insbesondere wird dafür gesorgt, daß auf einer Mikroelektrode nur eine Zelle zu liegen kommt. Andererseits bleibt es nach wie vor dem Zufall überlassen, ob eine Zelle und welche Zelle in der Mikroküvette eingefangen wird. Dabei ist ferner nicht auszuschließen, daß in der Flüssigkeit befindliche Partikel in die Mikroküvette eingesogen werden, die zwar die Durchführung einer Messung ermöglichen, deren Ergebnisse jedoch nicht verwertbar sind.

Ein weiterer - Nachteil bei der bekannten Vorrichtung und dem bekannten Verfahren besteht darin, daß nur Messungen an der Außenseite der Zellmembran durchgeführt werden können. Intrazelluläre Messungen sind nicht möglich.

Derartige intrazelluläre Messungen lassen sich jedoch mit der sogenannten Patch-Clamp-Technik durchführen, bei der eine sogenannte Membranfleck-Klemme eingesetzt wird; siehe z.B. Neher und Sakmann: "Die Erforschung von Zellsignalen mit der Patch-Clamp-Technik", Spektrum der Wissenschaften, Mai 1992, S. 48.

Bei dieser Technik wird eine mit elektrisch leitender Flüssigkeit gefüllte Mikropipette gezielt an eine adhärente Zelle herangeführt und auf deren Membran aufgesetzt, so daß sich diese leicht nach innen wölbt. Daraufhin wird der von der Pipettenspitze eingeschlossene Membranfleck durch Unterdruck leicht angesogen, wodurch dieser gegenüber der umgebenden Flüssigkeit abgedichtet und elektrisch isoliert wird. Diese Isolation wird auch als "Gigaseal" bezeichnet.

Auf diese Weise ist es möglich, z.B. Messungen an Ionenkanälen auf der Membranaußenseite durchzuführen.

Wenn die abgedichtete Membran jedoch weiter angesaugt wird, wird der Membranfleck perforiert, so daß ein hydrodynamisch und elektrisch gegenüber der umgebenden Flüssigkeit abgedichteter Zugang durch die Pipettenöffnung zum Zellinneren besteht, so daß intrazelluläre Messungen und Stimulationen möglich sind (sogenannter "Whole-Cell-Patch").

Bei der Patch-Clamp-Technik ist von Nachteil, daß sie nur an adhärenten oder anderweitig immobilisierten Zellen durchgeführt werden kann, an die die fragile Glaspipette jeweils mittels eines Mikromanipulators herangeführt werden muß. Aus diesem Grund ist die Anzahl gleichzeitig kontaktierbarer Zellen äußerst beschränkt, so daß mehrere Zellen nur sequentiell abgearbeitet werden können.

Diese Methode ist daher ungeeignet für Massenuntersuchungen, wie sie z.B. im Bereich des Pharmascreening, des Substanzscreening etc. erforderlich wären.

Ein weiterer Nachteil bei der konventionellen Patch-Clamp-Technik besteht darin, daß sie nicht automatisierbar ist, sondern manuell von Personal durchgeführt wird, das große Erfahrung mitbringen und viel Fingerspitzengefühl aufweisen muß.

Aus der DE 198 27 957 A1 ist es bekannt, eine Zelle auf einem Glasträger zu immobilisieren, dessen Oberfläche eine ringstrukturförmige Profilierung aufweist, um die Zelladhäsion zu verbessern. Auf dem Glasträger ist im Bereich der Auflagefläche für die Zelle eine kegelförmig vorspringende Elektrode angeordnet, die mit ihrer scharfen, ringförmigen Spitze in die Zellmembran einschneidet und für intrazelluläre Messungen vorgesehen ist. Die Elektrode kann als Zylinderelektrode mit einem Hohlkanal ausgelegt sein, durch den auf die Zellmembran ein Unterdruck zum Fixieren der Zelle und/oder Perforieren der Zellmembran ausgeübt werden kann.

Wie die Zelle auf den Auflagebereich gelangt, ist nicht beschrieben. Nachteilig ist bei der bekannten Vorrichtung, daß die elektrische Abdichtung auf der ringförmigen Elektrodenspitze nur durch die "Haltekraft" der Zelle auf der profilierten Oberfläche bestimmt wird. Die Durchführung von Massenuntersuchungen ist mit der bekannten Vorrichtung nicht möglich.

Die eingangs genannte WO 00/34776 schlägt vor, zu untersuchende Zellen an einer Flüssigkeits-/Luft-Grenzschicht zu immobilisieren und anschließend in Patch-Clamp-Manier zu kontaktieren. Dabei sollen ggf. mehrere Zellen mit Hilfe von arrayartig angeordneten Pipetten kontaktiert werden. Wie bei der klassischen Patch-Clamp-Technik müssen die Pipetten jedoch relativ zu den immobilisierten Zellen bewegt werden.

Vor diesem Hintergrund ist es eine Aufgabe der vorliegenden Erfindung, ein alternatives Verfahren sowie eine entsprechende Vorrichtung bereitzustellen, mit denen einzelne Zellen in Patch-Clamp-Manier kontaktiert werden können, wobei das Verfahren bzw. die entsprechende Vorrichtung eine weitgehend automatisierte Untersuchung von Zellen ermöglichen sollen.

Diese Aufgabe wird bei der eingangs genannten Vorrichtung dadurch gelöst, daß die Mittel zum Immobilisieren einen in die Öffnung mündenden Ansaugkanal beinhalten, über den an der Öffnung zum Immobilisieren der Zelle ein zweiter hydrodynamischer Unterdruck erzeugbar ist, wobei die Kontaktspitze so angeordnet ist, daß deren oberes Ende in die Öffnung hineinragt und mit einer Zellmembran der immobilisierten Zelle in Anlage gelangt.

Die Aufgabe wird ferner durch ein Verfahren der eingangs genannten Art gelöst, bei dem die Zelle durch Erzeugen eines zweiten hydrodynamischen Unterdrucks in einem Ansaugkanal auf der Öffnung immobilisiert wird, wobei der erste und der zweite hydrodynamische Unterdruck getrennt gesteuert werden.

Die der Erfindung zugrunde liegende Aufgabe wird auf diese Weise vollkommen gelöst.

Die Immobilisierung der Zellen auf der Kontaktspitze bewirkt dabei gleichzeitig die Wölbung der Zellmembran nach innen. Die Mittel zur Immobilisierung, vorzugsweise der Unterdruck in dem Ansaugkanal bewirken also neben der Immobilisierung auch den Kontakt zwischen Zellmembran und Kontaktspitze und führen ferner dazu, daß die Zellmembran auf die Kontaktspitze Druck ausübt. In die Öffnung ragt eine Patch-Clamp-Spitze hinein, über die außen an der Membran und/oder intrazellulär elektrische Signale gemessen und/oder zugeführt werden können. Dabei ist der durch die Mittel zum Immobilisieren bewirkte Haltedruck unabhängig und getrennt von dem Kontaktdruck, so daß die Kontaktspitze einen sehr kleinen Durchmesser aufweisen kann, der einen zuverlässigen Gigaseal und eine gute Perforation ermöglicht. Der Durchmesser der Öffnung ist deutlich größer als der Durchmesser der Kontaktspitze, so daß sich ein sehr großer Haltedruck ergibt, ohne daß die Zelle in den Ansaugkanal oder gar in den Kontaktkanal eingezogen wird.

Auf diese Weise sind stabile Langzeitmessungen auch unter Perfusion möglich, wenn die Zelle außen mit Substanzen angespült wird, um bestimmte Reaktionen der Zelle hervorzurufen, die wiederum über die Elektrode erfaßt werden sollen.

Weil die Kontaktspitze in die Öffnung hineinragt, ergibt sich beim Ansaugen der Zelle auf die Öffnung mittels des Ansaugkanals eine Einwölbung der Zellmembran im Bereich der Kontaktspitze, wie es auch bei der manuellen Patch-Clamp-Technik wünschenswert ist, um für einen guten Gigaseal zu sorgen, wenn durch den Kontaktkanal ein leichter Unterdruck auf den Membranfleck ausgeübt wird. Durch kurzfristiges, stoßweises Erhöhen des Unterdrucks im Kontaktkanal läßt sich in an sich bekannter Weise der Membranfleck jetzt reproduzierbar perforieren.

Die Erfinder der vorliegenden Anmeldung haben erkannt, daß durch die Trennung von Ansaugkanal und Kontaktkanal eine zuverlässige und damit automatisierbare Positionierung von Zellen über der Öffnung und deren Kontaktierung nach der Patch-Clamp-Technik möglich wird.

In einer Weiterbildung der erfindungsgemäßen Vorrichtung ist es bevorzugt, wenn die Kontakteinheit abnehmbar an der Öffnung angeordnet ist, wobei weiter vorzugsweise die Kontaktspitze auswechselbar angeordnet ist.

Hier ist von Vorteil, daß die Kontakteinheit und/oder die Kontaktspitze leicht ausgewechselt werden können, während die übrigen Bestandteile der neuen Vorrichtung wieder verwendbar sind. Dieser Maßnahme liegt die Erkenntnis zugrunde, daß Patch-Clamp-Spitzen nur einmal verwendet werden können, bereits bei einem zweiten Einsatz wird kein ausreichender Gigaseal bzw. keine zuverlässige Membranperforation mehr erreicht.

Dabei ist es bevorzugt, wenn der Ansaugkanal in dem Substrat verläuft, wobei andererseits der Ansaugkanal auch zumindest abschnittsweise in der Kontakteinheit verlaufen kann.

Wenn der Ansaugkanal in dem Substrat verläuft, ist von Vorteil, daß die Kontakteinheit sehr einfach und preiswert aufgebaut sein kann. Verläuft der Ansaugkanal dagegen zumindest abschnittsweise in der Kontakteinheit, so kann der Außenanschluß sowohl des Ansaugkanals als auch des Kontaktkanals auf einfache Weise an der Kontakteinheit selbst erfolgen, was das Handling und den Aufbau vereinfacht.

Weiter es ist.bevorzugt, wenn die Kontakteinheit ein austauschbares, die Kontaktspitze aufweisendes Oberteil sowie ein wiederverwendbares, die Elektrode und einen ersten Abschnitt des Kontaktkanals aufweisendes Unterteil aufweist, wobei die Kontakteinheit vorzugsweise im Bereich der Öffnung außen an dem Substrat anliegt und die Öffnung nach außen flüssigkeitsdicht abschließt, wobei weiter vorzugsweise das Oberteil unter Druck außen an dem Substrat und das Unterteil unter Druck an dem Oberteil anliegt.

Bei diesen Maßnahmen ist von Vorteil, daß zum Auswechseln der Kontaktspitze lediglich das Oberteil der Kontakteinheit ausgewechselt werden muß. Weil Unterteil und Oberteil aneinander bzw. an dem Substrat unter Druck anliegen, sind keine Schraubarbeiten oder ähnliche Manipulationen erforderlich, der Austausch kann automatisiert z.B. mit Hilfe eines Mikromanipulators auf einfache Weise erfolgen.

Dabei ist es bevorzugt, wenn zwischen dem Substrat und dem Oberteil und/oder zwischen dem Oberteil und dem Unterteil ein Dichtmittel vorgesehen ist, das vorzugsweise ausgewählt ist aus der Gruppe: Silikonpaste, Zwei-Komponenten-Elastomer und Polydimethylsiloxan-Elastomer (Sylgard^{™}).

Bei dieser Maßnahme ist von Vorteil, daß schon bei geringem Druck eine gute flüssigkeitsdichte Abdichtung zwischen Substrat, Oberteil und Unterteil erfolgt, so daß an den Mikromanipulator sowohl hinsichtlich Genauigkeit als auch hinsichtlich Druckausübung geringere Anforderungen zu stellen sind, als wenn bspw. ein O-Ring zur Abdichtung verwendet würde.

Weiter ist es bevorzugt, wenn in dem Unterteil eine Kammer vorgesehen ist, in die die Elektrode hineinragt und der erste Abschnitt des Kontaktkanals mündet.

Hier ist von Vorteile daß die Elektrode und der erste Abschnitt des Kontaktkanals in dem wiederverwendbaren Unterteil angeordnet sind, so daß die Kosten für die Durchführung einer Messung nur durch das austauschbare Oberteil bestimmt werden, daß lediglich die Kontaktspitze sowie einen zweiten Abschnitt des Kontaktkanals trägt.

In diesem Zusammenhang ist es bevorzugt, wenn in dem Oberteil ein der Kammer zugeordneter, die Kontaktspitze durchsetzender trichterförmiger zweiter Abschnitt des Kontaktkanals angeordnet ist.

Weiter ist es bevorzugt, wenn zwischen dem Oberteil und dem Substrat um die Kontaktspitze herum ein Ring ausgebildet ist, der auf das Unterteil zuweisend einen Durchbruch aufweist, der einem ersten Abschnitt des Ansaugkanals in dem Unterteil zugeordnet ist.

Auf diese einfache Weise kann der Ansaugkanal in die Kontakteinheit integriert werden. Der Ansaugkanal läuft jetzt von dem Unterteil über den Durchbruch in den Ringraum um die Kontaktspitze und saugt dort die Zelle auf die Öffnung und gegen die Kontaktspitze. Auch hier ist weiter von Vorteil, daß nur ein sehr einfach aufgebautes Oberteil, das wie alle anderen Komponenten in Mikrosystemtechnik, also mikrostrukturiert hergestellt sein kann, ausgetauscht werden muß.

In einer Weiterbildung ist es bevorzugt, wenn der Ringraum auf das Substrat zuweisend mit einer Membran abgedichtet ist, in der ein die Kontaktspitze mit Abstand umgebender Durchlaß vorgesehen ist.

Hier ist von Vorteil, daß die Öffnung in dem Substrat deutlich größer sein kann als der Durchlaß, durch den der Ansaugkanal die Zelle auf die Öffnung saugt. Auf diese Weise sind an die Manipuliergenauigkeit des Mikromanipulators geringere Anforderungen zu stellen, da nur sichergestellt werden muß, daß der Durchlaß im Bereich der Öffnung angeordnet ist, die Abdichtung nach außen erfolgt durch die Membran, die vorteilhafterweise an dem Oberteil befestigt ist.

Allgemein ist es bevorzugt, wenn im Bereich der Öffnung eine Referenzelektrode in die Flüssigkeit hineinragt.

Bei dieser Maßnahme ist von Vorteil, daß durch die räumliche Nähe von Elektrode und Referenzelektrode sehr zuverlässig und reproduzierbar Potentiale auf der Zellmembran oder intrazellulär gemessen werden können.

Allgemein ist es bevorzugt, wenn auf dem Substrat um die Öffnung herum eine funktionelle Beschichtung als Mittel zur Immobilisierung der Zellen vorgesehen ist.

Diese Maßnahme dient in vorteilhafter Weise der Selektion der zu kontaktierenden Zellen. Durch den Einsatz einer funktionellen Beschichtung ist es jetzt möglich, aus einer Suspension verschiedener Zellen eine bestimmte Art von Zellen zu selektieren und zu kontaktieren. Nur die Zellen, die an der auf die Zellen selbst abgestimmten funktionellen Beschichtung immobilisiert werden, werden auch zur Messung herangezogen.

Die funktionelle Beschichtung ist dabei ausgewählt aus der Gruppe: Polyanionen; Polykationen; Polyethylenimin; Antikörper gegen Zelloberflächenmoleküle wie Integrine (z.B. Fibronektin) oder Lektine; und magnetische Beschichtung.

Durch die Wahl der Polyanionen, Polykationen und Polyimine kann für eine geeignete chemische Immobilisierung gesorgt werden, während durch Immobilisierung geeigneter Antikörper in der funktionellen Beschichtung eine Spezifität für beliebige Zelloberflächenmoleküle hergestellt werden kann.

Wenn die funktionelle Beschichtung eine magnetische Beschichtung umfaßt, so lassen sich auf diese Weise magnetische Antikörper einfangen, die der Zellsuspension zuvor zugegeben wurden und sich an bestimmte Zelloberflächenmoleküle angelagert haben. Durch die Wahl eines speziellen magnetischen Antikörpers können auf diese Weise genau vorherbestimmbare Zellen aus der Zellsuspension immobilisiert werden. An magnetische Partikel gekoppelte Antikörper sind aus dem Stand der Technik bekannt, sie werden beispielsweise von Per Septive Diagnostics Incorporated, Cambrigde, Massachusetts, vertrieben; siehe Ahern: "Antibodies Making Their Way From The Clinic To The Research Lab", The Scientist, Band 9, 1995, S. 18-19.

Die Mittel zum Immobilisieren haben wie bspw. der Ansaugkanal ferner die Wirkung, daß die Immobilisierung selbst zum Kontakt zwischen Zellmembran und Kontaktspitze führt und auf die Zellmembran eine auf die Kontaktspitze gerichtete Kraft ausgeübt wird.

Dabei ist es weiter bevorzugt, wenn eine Positioniereinheit vorgesehen ist, um eine Zelle über der Öffnung zu positionieren, wobei die Positioniereinheit eine Laserpinzette umfassen kann, ein elektromagnetisches Feld aufbauen oder eine hydrodynamische Kraft erzeugen kann.

Die Positioniereinheit kann damit einerseits bereits durch den Ansaugkanal repräsentiert sein, der eine hydrodynamische Kraft erzeugt, wobei aber auch unabhängig von dem Ansaugkanal oder zusätzlich zu dem Ansaugkanal z.B. eine Laserpinzette oder ein elektromagnetisches Feld vorgesehen sein können.

Laserpinzetten sind aus dem Stand der Technik allgemein bekannt, bei diesen optischen Pinzetten handelt es sich um optische Manipulatoren, die auf dem Impulserhaltungssatz basieren.

Ein für das Laserlicht weitgehend transparentes Objekt absorbiert kaum Energie, lenkt den Strahl jedoch nach dem Brechungsgesetz aus der ursprünglichen Richtung ab. Diese Brechung überträgt auf eine Zelle einen Impuls, der die Zelle bewegt. Ein Paar symmetrischer Laserstrahlen bildet so eine Falle, in der die Zelle gehalten wird. Durch Verfahren der Strahlen läßt_sich die Zelle bewegen; siehe Berns: "Mit Laserwerkzeugen ins Zellinnere", Spektrum der Wissenschaften, Juli 1998, S. 56.

Mittels der Positioniereinheit kann z.B. eine Zelle auf die funktionelle Beschichtung aufgesetzt werden. Wird die Zelle danach von der Positioniereinheit freigegeben, so verbleibt sie nur über der Öffnung, wenn sie durch die funktionelle Beschichtung immobilisiert werden kann. Auf diese Weise läßt sich eine einfache Selektion von Zellen vornehmen. Wenn die Zelle von der Transportströmung in der Vorrichtung nach dem Freigeben durch die Positioniereinheit weiterbewegt, hat eine negative Selektion stattgefunden, andernfalls eine positive Selektion.

Auf diese Weise lassen sich aus einer Suspension unterschiedlicher Zellen genau die Zellen heraussuchen, die mit der Vorrichtung kontaktiert werden sollen. Auch diese Selektion ist bei einer gattungsgemäßen Vorrichtung und einem gattungsgemäßen Verfahren für sich genommen neu und erfinderisch.

Dabei ist es bevorzugt, wenn das Substrat in einem Strömungskanal angeordnet, vorzugsweise integraler Bestandteil einer Wandung eines Strömungskanals ist, durch den die Flüssigkeit mit definierter und/oder einstellbarer Transportströmung zu der Öffnung geleitet wird, wobei vorzugsweise eine Sensoreinheit vorgesehen ist, die auf einen Parameter der Zelle anspricht und die Positioniereinheit und/oder den Ansaugkanal ansteuert.

Das Substrat kann z.B. senkrecht zur Wandung des Strömungskanals und damit senkrecht zur Strömung stehen, so daß es von dieser direkt angeströmt wird.

Auch die Sensoreinheit dient der Selektion von Zellen und ist für sich genommen genauso wie die funktionelle Beschichtung bei einer Vorrichtung und einem Verfahren der gattungsgemäßen Art neu und erfinderisch.

Der Parameter ist dabei ausgewählt aus der Gruppe: optische Parameter wie beispielsweise Fluoreszenz, Absorption, Reflexion, Beugung, Brechung; geometrische Parameter wie beispielsweise Größe, Länge, Durchmesser, Form; elektrische Parameter wie beispielsweise Kapazität, Widerstand; und biologische, chemische, physikalische Zellparameter.

Die Sensoreinheit ist dabei vorzugsweise stromaufwärts von der Öffnung angeordnet.

Die Sensoreinheit ermöglicht insbesondere im Zusammenhang mit der einstellbaren Transportströmung und/oder der Positioniereinheit eine Selektion von Zellen. Wenn die Sensoreinheit anhand der gemessenen Zellparameter eine zu kontaktierende Zelle erkennt, wird dies an die Positioniereinheit und/oder den Ansaugkanal weitergegeben, die dann in Kenntnis der eingestellten Transportströmung zu dem Zeitpunkt tätig werden, zu dem sich die von der Sensoreinheit selektierte Zelle in ihrem Bereich befindet. Es ist auch möglich, kurz vor dem Ansaugkanal eine weitere Sensoreinheit vorzusehen, die zur Lokalisierung einer Zelle dient und deren momentanen Ort an den Ansaugkanal und/oder die Positioniereinheit meldet.

Die Sensoreinheit sowie die gegebenenfalls vorgesehene weitere Sensoreinheit ermöglichen damit eine deutlich effizientere Selektion von Zellen als die funktionelle Beschichtung, wobei durch Kombination von Sensoreinheit(en), Positioniereinheit und funktioneller Beschichtung eine sehr scharfe Selektion der zu kontaktierenden Zellen möglich wird.

Allgemein ist es bevorzugt, wenn stromaufwärts von der Öffnung eine Mikroperfusionseinheit angeordnet ist, um eine immobilisierte Zelle mit Testsubstanzen zu umspülen, wobei vorzugsweise die Komponenten der Vorrichtung in Mikrosystemtechnik hergestellt sind.

Auf diese Weise ist es möglich, die immobilisierte Zelle mit geringen Substanzmengen anzuspülen, so daß kostensparend gearbeitet werden kann.

Bei dem neuen Verfahren ist es bevorzugt, wenn vor jedem Kontaktieren die Kontaktspitze ausgetauscht wird.

Hier ist von Vorteil, daß für jedes neue Kontaktieren eine neue Kontaktspitze verwendet wird, was für die sichere Ausbildung eines Gigaseals sowie eine zuverlässige Perforation des umschlossenen Membranflecks sorgt, sofern dies gewünscht ist.

Weiter ist es bevorzugt, wenn die Flüssigkeit mit einstellbarer Transportströmung durch einen Kanal zu der Öffnung geleitet wird.

Hier ist von Vorteil, daß die suspendierten Zellen in bekannter Zeitabfolge an der Öffnung vorbeischwimmen, so daß eine Ablaufsteuerung den Ansaugkanal jeweils rechtzeitig ansteuern kann.

Weiter ist es bevorzugt, wenn die Zelle durch Anhaften an einer funktionellen Beschichtung immobilisiert wird, die um die-Öffnung herum auf dem Substrat angeordnet ist, wobei die Zelle vorzugsweise durch eine Positioniereinheit über der Öffnung positioniert wird und weiter vorzugsweise vor dem Positionieren einer Zelle ein Parameter der Zelle gemessen und in Abhängigkeit von dem Parameter die Zelle für die Positionierung ausgewählt wird.

Hier ist von Vorteil, daß nicht mit völlig reinen Zellpopulationen gearbeitet werden muß, sondern daß auch aus gemischten Zellsuspensionen gewünschte Zelltypen zur Kontaktierung ausgewählt werden können.

Dabei ist es bevorzugt, wenn eine Zelle dadurch selektiert wird, daß sie mittels der Positioniereinheit auf der funktionellen Beschichtung positioniert und dann von der Positioniereinheit freigegeben wird, so daß nur Zellen immobilisiert bleiben, die auf die funktionelle Beschichtung ansprechen.

Hier ist von Vorteil, daß quasi jede an der Positioniereinheit vorbeischwimmende Zelle über die funktionelle Beschichtung daraufhin getestet wird, ob sie kontaktiert werden soll oder aber freigegeben werden kann.

Dabei ist es bevorzugt, wenn die Zelle zusätzlich zu der funktionellen Beschichtung durch hydrodynamischen Unterdruck immobilsiert wird.

Diese Maßnahme hat den Vorteil, daß eine zuverlässie Haltekraft auf eine über die funktionelle Beschichtung selektierte Zelle ausgewählt wird, so daß sie während einer Langzeitmessung z.B. mit Perfusion sicher gehalten werden kann.

Schließlich ist es noch bevorzugt, wenn die Zelle von der Öffnung entfernt wird, indem der zur Immobilisierung angelegte Unterdruck zu einem Überdruck aufgebaut wird.

Hier ist von Vorteil, daß nach dem Ende einer Meß- und Stimulationsphase die Zelle aktiv wieder abgestoßen werden kann.

Zusammenfassend bieten die neue Vorrichtung sowie das neue Verfahren also die Möglichkeit, Einzelzellen aus einer Suspension nach mindestens einem Kriterium, also einem Zellparameter, zu selektieren und die selektierten Einzelzellen aus der Suspension an einer Ableitstelle, also an der Öffnung, zu positionieren und zu immobilisieren. Die Immobilisierung erfolgt dabei entweder über einen Ansaugkanal, also mit hydrodynamischem Unterdruck, und/oder aber über eine funktionelle Beschichtung.

Entsprechend dem Verfahren der klassischen Patch-Clamp-Technik wird die Zelle zum Zwecke der Bildung eines hochomigen Seals durch den Kontaktkanal angesaugt, wobei die angesaugte und abgedichtete Membran wahlweise durch einen Druckpuls durchbrochen werden kann.

Die immobilisierte und kontaktierte Zelle kann mit Flüssigkeiten perfundiert werden, die Testsubstanzen enthalten, so daß serielle Untersuchungen an vielen Zellen automatisierbar durchgeführt werden können, wobei gleichzeitig die Vorteile der klassischen Patch-Clamp-Technik genutzt werden.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Übersicht über die neue Vorrichtung;
- Fig. 2: eine vergrößerte Darstellung eines Ausschnitts der Vorrichtung aus Fig. 1 im Bereich der Kontaktier-und Meßeinheit;
- Fig. 3: den schematischen Ablauf einer elektrischen Kontaktierung einer Zelle sowie des Austauschs der Kontaktspitze;
- Fig. 4: ein erstes Ausführungsbeispiel einer austauschbaren Kontaktspitze, bei der der Ansaugkanal im Substrat angeordnet ist;
- Fig. 5: ein Ausführungsbeispiel wie Fig. 4, bei dem jedoch die Kontakteinheit in auswechselbares Oberteil und wiederverwendbares Unterteil aufgeteilt ist;
- Fig. 6: eine Kontakteinheit wie in Fig. 5, wobei jedoch der Ansaugkanal in der Kontakteinheit ausgebildet ist; und
- Fig. 7: eine Kontakteinheit wie in Fig. 6, jedoch mit größerer Öffnung im Substrat.

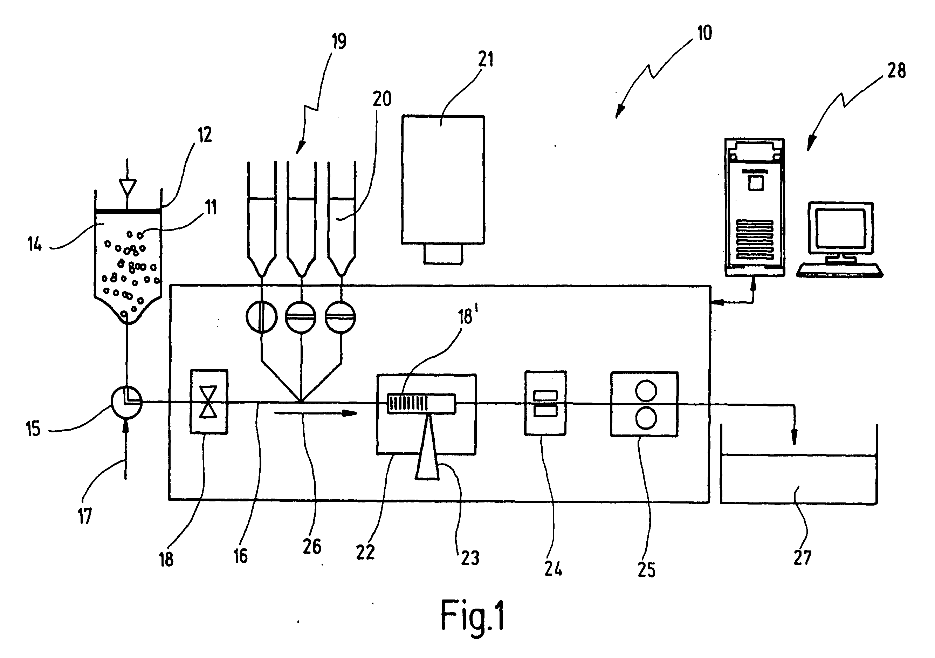

In Fig. 1 ist mit 10 eine Vorrichtung zum elektrischen Kontaktieren von Zellen 11 gezeigt, die in einer in einem Vorratsbehälter 12 vorrätig gehaltenen Flüssigkeit 14 in Suspension sind.

Der Vorratsbehälter 12 ist mit einem Umschaltventil 15 verbunden, durch das die Flüssigkeit 14 in einen Strömungskanal 16 geleitet wird, in dem in noch zu beschreibender Weise die elektrische Kontaktierung der Zellen 11 erfolgt.

An das Umschaltventil 15 ist ferner ein Einlaß 17 für eine Waschlösung angeschlossen, so daß der Strömungskanal 16 nach erfolgter Messung an einer Zelle 11 mit der Waschlösung durchspült werden kann.

Längs des Strömungskanals 16 ist zunächst eine Sensoreinheit 18 angeordnet, über die Parameter der Zellen 11 gemessen werden.

An die Sensoreinheit 18 schließt sich eine Mikroperfusionseinheit 19 an, über die Perfusionslösungen 20 in den Strömungskanal 16 eingeleitet werden können, um eine kontaktierte Zelle zu perfundieren, also mit Testsubstanzen anzuspülen.

An die Mikroperfusionseinheit 19 schließt sich eine Positioniereinheit 21 an, über die Zellen 11 in einer Kontaktier- und Meßeinheit 22 positioniert und dort immobilisiert werden können.

Die Positioniereinheit 21 wird z.B. in Abhängigkeit von Ausgangssignalen der Sensoreinheit 18 gesteuert.

In Fig. 1 ist in der Kontaktier- und Meßeinheit 22 noch eine konventionelle Patch-Clamp-Spitze 23 angedeutet, über die Messungen an der Außenseite einer Membran der Zelle 11 sowie intrazelluläre Messungen in noch zu beschreibender Weise automatisch durchgeführt werden können.

Vor der Patch-Clamp-Spitze 23 kann eine weitere Sensoreinheit 18' angeordnet sein, die bei dem in Fig. 1 gezeigten Ausführungsbeispiel in der Kontaktier- und Meßeinheit 22 angeordnet ist. Die weitere Sensoreinheit 18' kann über optische und andere Methoden Einzelzellen im Strömungskanal 16 lokalisieren und mit ihrem Meßkanal das Ansaugen dieser Einzelzelle steuern, wie es im einzelnen weiter hinten noch beschrieben wird.

Während die Sensoreinheit 18 zur Selektion einer anzusaugenden Zelle dient, ist es Aufgabe der weiteren Sensoreinheit 18', eine Einzelzelle zu lokalisieren und deren momentanen Ort derart zu bestimmen, daß sie über die Positioniereinheit 21 und/oder durch Ansaugen in Kontakt mit der Patch-Clamp-Spritze 23 gebracht werden kann.

An die Kontaktier- und Meßeinheit 22 schließt sich eine zweite Sensoreinheit 24 und daran eine Steuerung 25 für eine bei 26 angedeutete Transportströmung der Flüssigkeit 14 in dem Strömungskanal 16 an. Der Strömungskanal 16 mündet schließlich in einen Auffangbehälter 27 für Flüssigkeit 14 und Waschlösung 17.

Schließlich ist in Fig. 1 noch eine Ablaufsteuerung und Meßdatenverarbeitung 28 gezeigt, über die die einzelnen Komponenten der Vorrichtung gesteuert und funktionell miteinander verbunden werden.

Über die Ablaufsteuerung und Meßdatenverarbeitung 28 wird die Transportströmung 26 dabei so eingestellt, daß jeweils eine einzige Zelle an der Sensoreinheit 18 vorbeiströmt, dort bezüglich bestimmter Parameter vermessen wird und dann im Falle ihrer Auswahl durch die Positioniereinheit 21 in der Kontaktier-und Meßeinheit positioniert wird.

Die Positioniereinheit 21 kann dabei z.B. eine Laserpinzette sein, oder aber ein elektromagnetisches Feld aufbauen, über das an magnetische Partikel gekoppelte Antikörper, die auf der Oberfläche der Zellen 11 sitzen, zur Meß- und Ableitstelle transportiert werden.

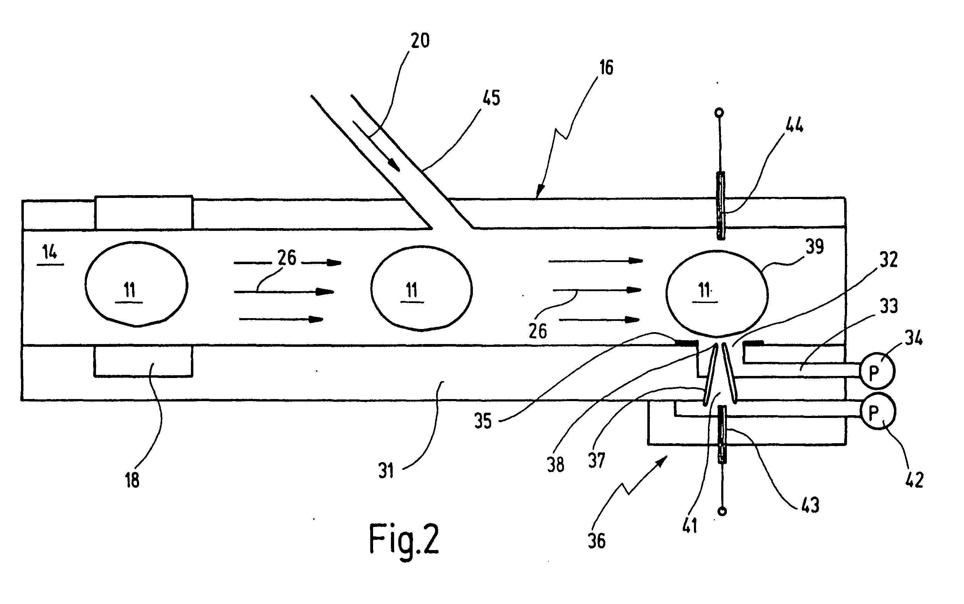

Darüber hinaus ist es auch möglich, statt oder zusätzlich zur Positioniereinheit 21 in der Kontaktier- und Meßeinheit 22 einen hydrodynamischen Unterdruck zum Immobilisieren einer Zelle 11 vorzusehen, wie dies nachstehend im Zusammenhang mit Fig. 2 beschrieben wird.

Die Sensoreinheit 18 ist dabei dazu ausgelegt, einen optischen, elektrischen, geometrischen oder sonstigen biologischen, chemischen oder physikalischen Zellparameter zu erfassen und an die Ablaufsteuerung und Meßdatenverarbeitung 28 zu melden, die daraufhin die Positioniereinheit 21 und/oder die Kontaktier- und Meßeinheit 22 entsprechend ansteuert, wenn die anhand ihres Parameters als geeignet erkannte Zelle aufgrund der bekannten Transportströmung 26 in den Bereich der Kontaktier- und Meßeinheit 22 gelangt ist.

Die Kontaktier- und Meßeinheit 22 ist dabei in Mikrosystemtechnik als mikrostrukturiertes Teil ausgelegt, weist also sehr geringe Abmaße auf.

In Fig. 2 ist ein Ausschnitt der Vorrichtung 10 aus Fig. 1 in vergrößertem Maßstab und in schematischer Seitenansicht gezeigt. Der Strömungskanal 16 weist als Wandung ein Substrat 31 auf, in dem eine Öffnung 32 vorgesehen ist, auf der eine zu kontaktierende Zelle 11 positioniert wird. In der Öffnung 32 mündet ein Ansaugkanal 33, der mit einer Pumpe 34 verbunden ist, über die in der Öffnung 32 zum Ansaugen einer Zelle 11 ein hydrodynamischer Unterdruck eingestellt werden kann.

Um die Öffnung 32 herum ist der Zelle 11 zugewandt auf dem Substrat 31 eine funktionelle Beschichtung 35 angeordnet, die zusätzlich zum Ansaugkanal 33 zum Immobilisieren einer Zelle 11 dient. Wenn die Zelle 11 auf der Öffnung 32 positioniert wurde, wird der Unterdruck der Pumpe 34 abgestellt, so daß die Zelle 11 nur auf der funktionellen Beschichtung 35 haften bleibt, wenn sie auf die Beschichtung anspricht, ansonsten wird sie durch die Transportströmung 26 weggewaschen. Für die eigentliche Messung wird die Pumpe 34 dann jedoch wieder eingeschaltet, so daß die Zelle 11 sicher auf der Öffnung 32 immobilisiert wird.

Die erste Positionierung auf der Öffnung 32 diente also lediglich dem Test, ob die Zelle 11 auf die funktionelle Beschichtung 35 anspricht, die für eine chemische Immobilisierung oder eine Immobilisierung über ihrerseits immobilisierte Antikörper sorgt. Die funktionelle Beschichtung 35 kann auch eine magnetische Beschichtung sein, die an magnetische Partikel gekoppelte Antikörper festhält, die ihrerseits auf der Zelle 11 sitzen.

Diese erste Positionierung der Zelle 11 auf der Öffnung 32 kann außer über den Ansaugkanal 33 auch über die Positioniereinheit 21 aus Fig. 1 erfolgen.

Von der Zelle 11 abgelegen sitzt an dem Substrat 31 eine abnehmbare Kontakteinheit 36, an der eine auswechselbare Kontaktspitze 37 angeordnet ist. Die Kontaktspitze 37 liegt mit ihrem oberen Ende 38 an einer Zellmembran 39 der Zelle 11 an und wölbt diese leicht nach innen.

Die Kontaktspitze 37 wird von einem Kontaktkanal 41 durchsetzt, der am oberen Ende 38 der Kontaktspitze 37 mündet. Anderen Endes ist der Kontaktkanal 41 mit einer Pumpe 42 verbunden, über die ein hydrodynamischer Unterdruck einstellbar ist.

In den Kontaktkanal 41 ragt eine Elektrode 43 hinein, die über in den Kontaktkanal 41 vorhandene elektrisch leitende Flüssigkeit mit der Zellmembran 39 in elektrischer Verbindung ist. Als Gegenelektrode dient eine Referenzelektrode 44, die in den Strömungskanal 16 im Bereich der Öffnung 32 hineinragt.

Durch den Ansaugkanal 33 wird die Zelle 11 nicht nur auf der Öffnung 32 immobilisiert, sondern mit ihrer Zellmambran 39 auf das obere Ende 38 der Kontaktspitze 37 gedrückt, während die Kontaktierung - wie bei der üblichen Patch-Clamp-Technik - durch Anlegen eines leichten Unterdrucks im Kontaktkanal 41 erfolgt. Wenn im Kontaktkanal 41 ein Druckstoß erzeugt wird, wird der von der Kontaktspitze 37 umschriebene Membranfleck perforiert, so daß intrazelluläre Messungen und Stimulierungen möglich werden.

In den Strömungskanal 16 mündet ferner noch ein Perfusionskanal 45, durch den eine Perfusionslösung 20 zu der immobilisierten und kontaktierten Zelle 11 geleitet werden kann, um diese mit Testsubstanzen zu umspülen und die Reaktion der Zelle 11 auf die Testsubstanzen zu messen.

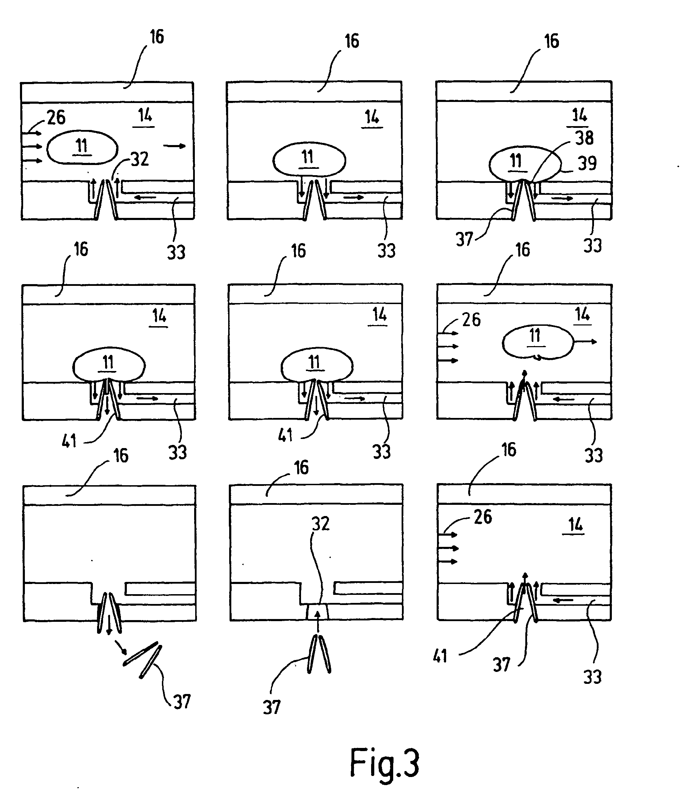

In Fig. 3 ist in einer schematischen Bildabfolge der Vorgang einer Kontaktierung und Messung/Stimulierung einer Zelle 11 gezeigt.

In dem ersten Bild in der oberen Reihe wird der Strömungskanal . 16 von einer Flüssigkeit 14 durchströmt, die eine Zelle 11 mit sich trägt. Durch den Ansaugkanal 33 wird ebenfalls Flüssigkeit 14 in das Innere des Strömungskanals 16 eingespült.

Wenn die Zelle 11 den Bereich der Öffnung 32 erreicht hat, wird über den Ansaugkanal 33 ein hydrodynamischer Unterdruck erzeugt, so daß die Zelle 11 auf die Öffnung 32 zugesogen wird, wie es im zweiten Bild der ersten Reihe gezeigt ist.

Das letzte Bild der ersten Reihe zeigt, daß die Zelle 11 mit ihrer Membran 39 nach innen gewölbt auf dem oberen Ende 38 der Kontaktspitze 37 zum Liegen kommt, wie es bei der klassischen Patch-Clamp-Technik auch der Fall ist. Im Gegensatz zur klassischen Technik erfolgt die Kontaktierung bei der neuen Vorrichtung und dem neuen Verfahren jedoch dadurch, daß die Zelle 11 zu der Kontaktspitze transportiert und dort automatisch immobilisiert und auf die Kontaktspitze 37 gedrückt wird.

In der zweiten Zeile im ersten Bild ist zu erkennen, daß ein Unterdruck an den Kontaktkanal 41 angelegt wird, so daß die immobilisierte Zelle in der in der Patch-Clamp-Technik üblichen Weise kontaktiert wird. Wenn der Unterdruck in dem Kontaktkanal 41 pulsartig erhöht wird, reist der Membranfleck auf, so daß eine intrazelluläre Messung möglich ist, wie es im zweiten Bild der zweiten Zeile gezeigt ist. Durch die Kombination von Ansaugdruck im Ansaugkanal 33 und Kontaktunterdruck im Kontaktkanal 41 ergibt sich zwischen Zellmembran 39 und oberem Ende 38 der Kontaktspitze 37 ein Gigaseal wie bei der klassischen Patch-Clamp-Technik. Allerdings wird der Gigaseal hier zwischen der Zellmembran 39 und der kleinen Stirnfläche des oberen Endes 38 und nicht einer großen Auflagefläche gebildet, wie es bei der eingangs erwähnten DE 198 27 957 A1 der Fall ist.

Wenn die Messung/Stimulierung beendet wurde, wird der Unterdruck im Kontaktkanal 41 abgeschaltet und im Ansaugkanal 43 wird ein Überdruck angelegt, so daß die Zelle 11 wieder in die Transportströmung 26 freigegeben und durch diese wegtransportiert wird.

In der dritten Zeile im linken Bild ist zu erkennen, daß danach die Kontaktspitze aus der Öffnung 32 entfernt und eine neue Kontaktspitze 37 eingesetzt wird, was im zweiten Bild der dritten Zeile angedeutet ist.

Nachdem die Kontaktspitze 37 ausgewechselt wurde, werden der Strömungskanal 16 sowie der Ansaugkanal 33 und der Kontaktkanal 41 zunächst mit Flüssigkeit durchspült, um das System für die Kontaktierung einer neuen Zelle 11 vorzubereiten.

Das Auswechseln der Kontaktspitze 37 erfolgt automatisch mittels eines Mikromanipulators, so daß mit der neuen Vorrichtung 10 automatisiert nacheinander viele Zellen kontaktiert und gemessen/stimuliert werden können.

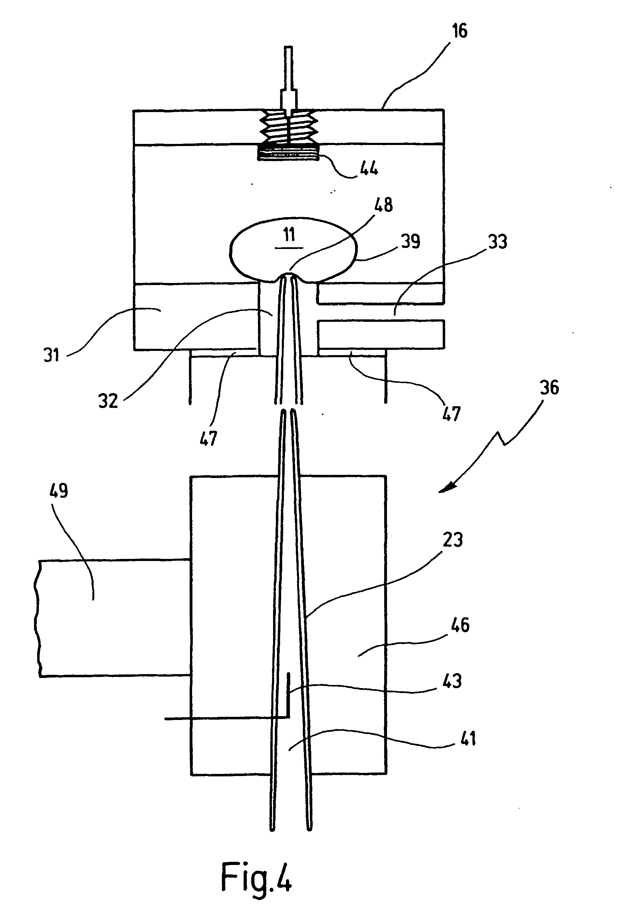

In Fig. 4 ist ein erstes, sehr einfach aufgebautes Ausführungsbeispiel für eine auswechselbare Kontaktspitze 37 gezeigt.

In dem gezeigten Ausführungsbeispiel ist die Kontaktspitze eine übliche Patch-Clamp-Spitze 23, wie sie aus Fig. 1 bereits bekannt ist. Die Patch-Clamp-Spitze 23 ist in einen Kunststoffblock 46 eingegossen, der von außen gegen das Substrat 31 gedrückt wird, so daß die Patch-Clamp-Spitze 23 in die Öffnung 32 hineinragt. Durch Dichtmittel 47, die vorzugsweise durch Sylgard^{™} gebildet werden, wird die Öffnung 32 flüssigkeitsdicht nach außen abgeschlossen.

Die Patch-Clamp-Spitze 23 ragt in dem gezeigten Ausführungsbeispiel etwas über das Substrat 31 hervor in den Strömungskanal 16 hinein, so daß eine über den Ansaugkanal 33 auf die Öffnung 32 gesaugte und gedrückte Zelle 11 eine bei 48 angedeutete Einwölbung ihrer Zellmembran 39 ausbildet, wie es auch von Hand durchgeführter Patch-Clamp-Technik eingestellt wird.

In Fig. 4 ist im oberen Bild die Anlage des Kunststoffblocks 46 an den Strömungskanal 16 gezeigt, während im unteren Bild der Kunststoffblock 46 mit in dem Kontaktkanal 41 angeordneter Elektrode 43 gezeigt ist. An dem Kunststoffblock 46 greift ein Mikromanipulator an, der lediglich schematisch dargestellt ist. Der Mikromanipulator 49 drückt den Kunststoffblock 45 gegen das Substrat 31 und hält den Kunststoffblock 46 unter Druck in Anlage mit dem Substrat 31, so daß der bereits erwähnte flüssigkeitsdichte Abschluß erreicht wird.

Die in Fig. 4 gezeigte Kontakteinheit 36 ist sehr einfach aufgebaut, sie besteht im wesentlichen aus einer konventionellen Patch-Clamp-Spitze 23, die in den Kunststoffblock 46 eingegossen wurde.

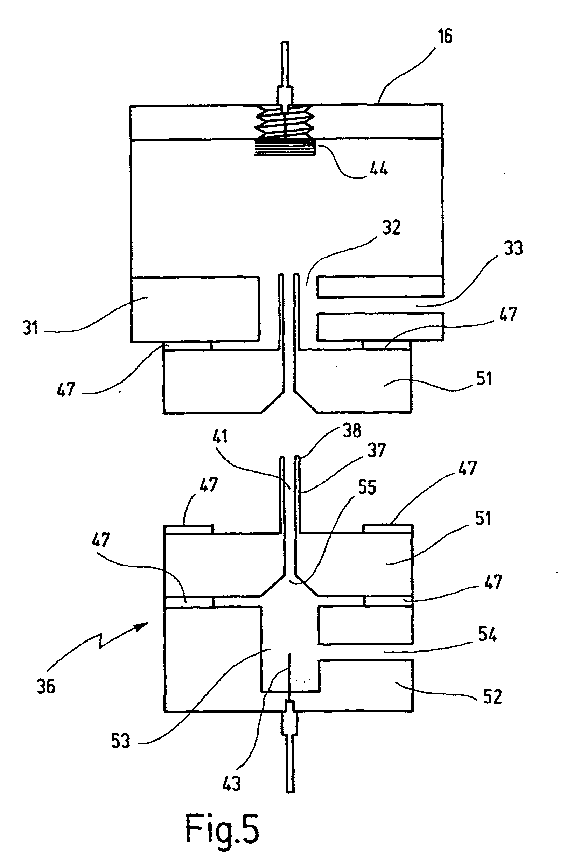

Ein weiteres Ausführungsbeispiel einer Kontakteinheit 36 ist in Fig. 5 gezeigt. Die Kontakteinheit 36 aus Fig. 5 weist ein auszuwechselndes Oberteil 51 sowie ein wiederverwendbares Unterteil 52 auf. An dem Oberteil 51 ist die Kontaktspitze 37 mit dem Kontaktkanal 41 angeordnet, wobei in dem Unterteil 52 eine Kammer 53 vorgesehen ist, in die die Elektrode 43 hineinragt. In die Kammer 53 führt ebenfalls ein erster Abschnitt 54 des Kontaktkanals 41, dessen zweiter, trichterförmiger Abschnitt 55 im Oberteil 51 angeordnet ist.

Zwischen Oberteil 51 und Unterteil 52 sitzen die schon aus Fig. 4 bekannten Dichtmittel 47, die auch zwischen Oberteil 51 und Substrat 31 angeordnet sind.

Die Kontakteinheit 36 aus Fig. 5 ist sehr einfach aufgebaut und leicht zu positionieren, wobei zum Auswechseln der Kontaktspitze 37 lediglich das Oberteil 51 ausgewechselt werden muß.

Hierzu wird über einen Mikromanipulator die gesamte Konakteinheit 36 von dem Substrat 31 entfernt, woraufhin das Oberteil 51 ausgetauscht und das Unterteil 52 mit neuem Oberteil 51 wieder gegen das Substrat 31 gedrückt wird. Dieses Auswechseln kann automatisch erfolgen, was gegenüber dem manuellen Austausch eine merkliche Zeitersparnis bringt.

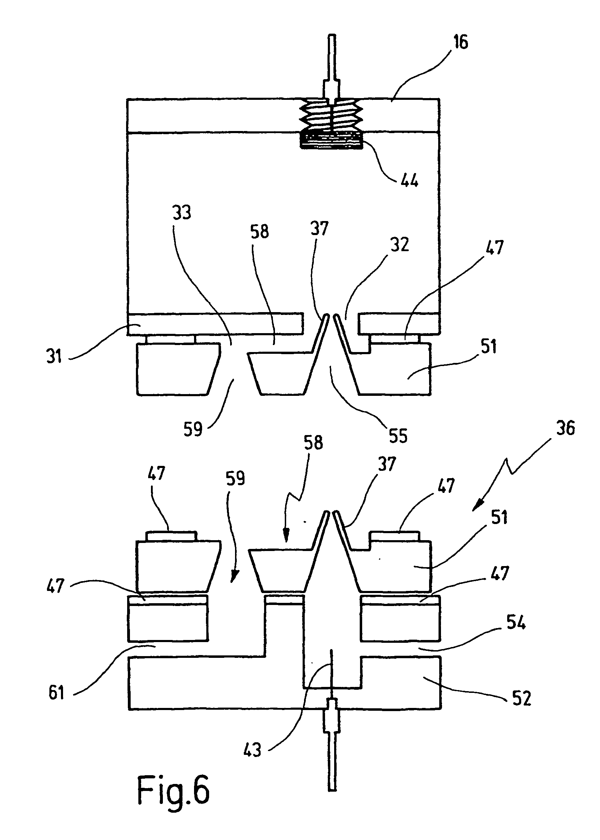

In dem Ausführungsbeispiel der Kontakteinheit 36 aus Fig. 6 ist zwischen dem Oberteil 51 und dem Substrat 31 ein Ringraum 58 ausgebildet, der die Kontaktspitze 37 umgibt. Der Ringraum 58 führt über einen Durchbruch 59 im Oberteil 51 zu einem ersten Abschnitt 61 des Ansaugkanals 33, der bei diesem Ausführungsbeispiel auch in der Kontakteinheit 36 angeordnet ist.

Wenn das Oberteil 51 gegen das Substrat 31 gedrückt wurde, wozu das Unterteil 52 gegen das Oberteil 51 gedrückt wird, sorgen die bereits bekannten Dichtmittel 47 wieder für einen flüssigkeitsdichten Abschluß.

Bei dem Ausführungsbeispiel gemäß Fig. 6 sind sämtliche hydrodynamischen Anschlüsse am Unterteil 52 vorgesehen, wobei in bekannter Weise der Ansaugkanal 33 in der Öffnung mündet.

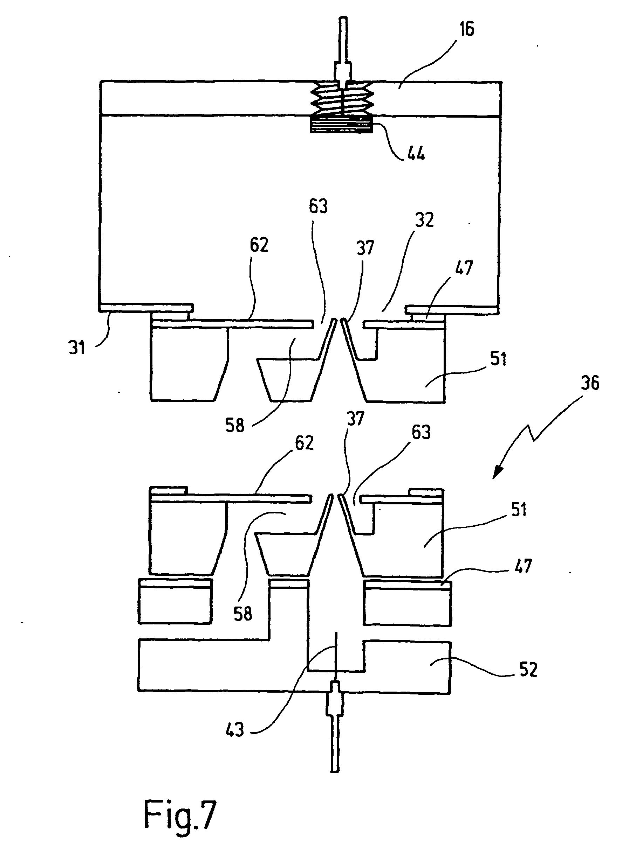

Bei dem Ausführungsbeispiel für die Kontakteinheit 36 in Fig. 7 ist gegenüber dem Ausführungsbeispiel der Fig. 6 der Ringkanal 58 zum hier sehr dünn ausgebildeten Substrat 31 hin durch-eine Membran 62 abgeschlossen, die um die Kontaktspitze 37 herum einen Durchlaß 63 bildet.

Im Vergleich zu Fig. 6 ist zu erkennen, daß die Öffnung 32 im Substrat 31 erheblich größer ist, so daß die Kontakteinheit 36 sehr leicht an dem Substrat 31 positioniert werden kann. Im übrigen entspricht die Kontakteinheit 36 aus Fig. 7 der Kontakteinheit 36 aus Fig. 6.

## Patentansprüche

1. Vorrichtung zum elektrischen Kontaktieren von in einer Flüssigkeit (14) in Suspension befindlichen biologischen Zellen (11), mit einem zumindest eine Öffnung (32) aufweisenden Substrat (31), mit Mitteln (33, 35) zum Immobilisieren einer Zelle (11) auf der Öffnung (32), und mit mindestens einer Elektrode (43) für die elektrische Kontaktierung einer immobilisierten Zelle (11), wobei die Elektrode (43) elektrisch mit einem in einer Kontaktspitze (37) mündenden Kontaktkanal (41) in Verbindung steht, über den auf die Zelle (11) ein erster hydrodynamischer Unterdruck ausübbar ist, **dadurch gekennzeichnet, dass** die Mittel (33, 35) zum Immobilisieren einen in die Öffnung (32) mündenden Ansaugkanal (33) beinhalten, über den an der Öffnung (32) zum Immobilisieren der Zelle (11) ein zweiter hydrodynamischer Unterdruck erzeugbar ist, wobei die Kontaktspitze (37) so angeordnet ist, dass deren oberes Ende (38) in die Öffnung (32) hineinragt und mit einer Zellmembran (39) der immobilisierten Zelle (11) in Anlage gelangt.

2. Vorrichtung nach Anspruch 1, **dadurch gekennzeichnet, dass** die Kontaktspitze (23, 37) auswechselbar angeordnet ist.

3. Vorrichtung nach Anspruch 1 oder 2, **dadurch gekennzeichnet, dass** der Ansaugkanal (33) in dem Substrat (31) verläuft.

4. Vorrichtung nach Anspruch 1 oder 2, **dadurch gekennzeichnet, dass** der Ansaugkanal (33) zumindest abschnittsweise in einer Kontakteinheit (36) verläuft, die ferner die Kontaktspitze (37) beinhaltet.

5. Vorrichtung nach Anspruch 4, **dadurch gekennzeichnet, dass** die Kontakteinheit ein austauschbares, die Kontaktspitze (37) aufweisendes Oberteil (51) sowie ein wiederverwendbares, die Elektrode (43) und einen ersten Abschnitt (54) des Kontaktkanals (41) aufweisendes Unterteil (52) aufweist.

6. Vorrichtung nach einem der Ansprüche 1 bis 5, **dadurch gekennzeichnet, dass** um die Kontaktspitze (37) herum ein Ringraum (58) ausgebildet ist, der dem Ansaugkanal (33) zugeordnet ist.

7. Vorrichtung nach einem der Ansprüche 1 bis 6, **gekennzeichnet durch** eine Positioniereinheit (21), um eine Zelle (11) über der Öffnung (32) zu positionieren.

8. Vorrichtung nach einem der Ansprüche 1 bis 7, **dadurch gekennzeichnet, dass** das Substrat (31) in einem Strömungskanal (16) angeordnet, vorzugsweise integraler Bestandteil einer Wandung eines Strömungskanals (16) ist, durch den die Flüssigkeit (14) mit definierter und/oder einstellbarer Transportströmung (26) zu der Öffnung (32) geleitet wird.

9. Vorrichtung nach einem der Ansprüche 1 bis 8, **gekennzeichnet durch** eine Sensoreinheit (18), die auf einen Parameter der Zellen (11) anspricht und eine Positioniereinheit (21) und/oder den Ansaugkanal (33) ansteuert, wobei die Sensoreinheit (18) vorzugsweise stromaufwärts von der Öffnung (32) angeordnet ist.

10. Vorrichtung nach Anspruch 8 oder 9, **dadurch gekennzeichnet, dass** stromaufwärts von der Öffnung (32) eine Mikroperfusionseinheit (19) angeordnet ist, um eine immobilisierte Zelle (11) mit Testsubstanzen zu umspülen.

11. Verfahren zum elektrischen Kontaktieren von in einer Flüssigkeit (14) in Suspension befindlichen biologischen Zellen (11) mit den Schritten:
- Immobilisieren einer Zelle (11) oberhalb einer in einem Substrat (31) vorgesehenen Öffnung (32), und
- Kontaktieren der immobilisierten Zelle (11) über zumindest eine Elektrode (43), wobei die Elektrode (43) durch eine Kontaktspitze (37) hindurch mit der Zelle (11) elektrisch in Kontakt gelangt, und wobei durch einen in der Kontaktspitze (37) mündenden Kontaktkanal (41) auf eine Zellmembran (39) der Zelle (11) ein erster hydrodynamischer Unterdruck ausgeübt wird,
**dadurch gekennzeichnet, dass** die Zelle (11) durch Erzeugen eines zweiten hydrodynamischen Unterdrucks in einem Ansaugkanal (33) auf der Öffnung (32) immobilisiert wird, wobei der erste und der zweite hydrodynamische Unterdruck getrennt gesteuert werden.

12. Verfahren nach Anspruch 11, **dadurch gekennzeichnet, dass** vor jedem Kontaktieren die Kontaktspitze (37, 23) ausgetauscht wird.

13. Verfahren nach Anspruch 11 und 12, **dadurch gekennzeichnet, dass** die Flüssigkeit (14) mit einstellbarer Transportströmung (26) durch einen Strömungskanal (16) zu der Öffnung (32) geleitet wird.

14. Verfahren nach Anspruch 11, **dadurch gekennzeichnet, dass** der von der Kontaktspitze (37) umschriebene Membranfleck der Zellmembran (39) der immobilisierten Zelle (11) mit Hilfe des ersten hydrodynamischen Unterdrucks perforiert wird.

## Claims

1. An apparatus for electrically contacting biological cells (11) suspended in a liquid (14), said apparatus comprising a substrate (31) having at least one opening (32), means (33, 35) for immobilizing a cell (11) on said opening (32), and at least one electrode (43) for electrically contacting an immobilized cell (11), whereby said electrode (43) is in electrical communication with a contact channel (41) which opens into a contact tip (37), via which contact channel a first hydrodynamic low pressure can be exerted upon said cell (11),
**characterized in that** said means (33, 35) for immobilizing comprise a suction channel (33) ending in said opening (32) via which suction channel a second hydrodynamic low pressure can be generated at the opening (32) to immobilize a cell on said opening (32), whereby said contact tip (37) is arranged such that its top end (38) projects into said opening (32) and comes to bear against a cell membrane (39) of an immobilized cell (11).

2. The apparatus of claim 1 **characterized in that** the contact tip (23, 37) is arranged such that it can be replaced.

3. The apparatus of claim 1 or 2, **characterized in that** the suction channel (33) runs inside the substrate (31).

4. The apparatus of claim 1 or 2, **characterized in that** at least some sections of the suction channel (33) run inside a contact unit (36) that further comprises said contact tip (37).

5. The apparatus of claim 4, **characterized in that** the contact unit has a replaceable top part (51) comprising the contact tip (37) and a reusable bottom part (52) comprising the electrode (43) and a first section (54) of the contact channel (41).

6. The apparatus of any of claims 1 to 5, **characterized in that** an annular space (58) is formed around the contact tip (37), which annular space is related to the suction channel (33).

7. The apparatus of any of claims 1 to 6, **characterized by** a positioning unit (21) for positioning a cell (11) above the opening (32).

8. The apparatus of any of claims 1 to 7, **characterized in that** the substrate (31) is arranged in a flow channel (16) and is preferably an integral part of a wall of said flow channel (16), through which flow channel the liquid (14) is directed to the opening (32) with a defined and/or adjustable transport flow (26).

9. The apparatus of any of claims 1 to 8, **characterized by** a sensor unit (18) which responds to a parameter of the cells (11) and which controls a positioning unit (21) and/or the suction channel (33), whereby the sensor unit (18) is arranged preferably upstream of the opening (32).

10. The apparatus of claim 8 or 9, **characterized in that** a microperfusion unit (19) is arranged upstream of the opening (32) for applying test substances to an immobilized cell (11).

11. A method for electrically contacting biological cells (11) suspended in a liquid (14), comprising the steps of:
- immobilizing a cell (11) above an opening (32) provided in a substrate (31), and
- contacting the immobilized cell (11) via at least one electrode (43), with the electrode (43) electrically contacting the cell (11) through a contact tip (37), and whereby through a contact channel (41) opening into said contact tip (37) a first hydrodynamic low pressure is exerted upon a cell membrane (39) of the cell (11),
**characterized in that** the cell (11) is immobilized upon said opening (32) by generating a second hydrodynamic low pressure in a suction channel (33), whereby said first and second hydrodynamic low pressure are controlled independently.

12. The method of claim 11, **characterized in that** the contact tip (37, 23) is replaced before each contacting.

13. The method of claims 11 and 12, **characterized in that** the liquid (14) is directed to the opening (32) with adjustable transport flow (26) through a flow channel (16).

14. The method of claim 11, **characterized in that** the membrane patch of the said membrane (39) of said immobilized cell (11) that is surrounded by said contact tip (37) is being perforated with the aid of said first hydrodynamic low pressure.

## Revendications

1. Dispositif de mise en contact électrique de cellules biologiques (11) en suspension dans un liquide (14), avec un substrat (31) présentant au moins un orifice (32), avec des moyens (33, 35) pour immobiliser une cellule (11) sur l'orifice (32), et avec au moins une électrode (43) pour la mise en contact électrique d'une cellule (11) immobilisée, dans lequel l'électrode (43) est reliée électriquement à un canal de contact (41) qui aboutit à une pointe de contact (37) et par le biais duquel une première dépression hydrodynamique peut être appliquée à la cellule (11), **caractérisé en ce que** les moyens d'immobilisation (33, 35) possèdent un canal d'aspiration (33) aboutissant à l'orifice (32) et par le biais duquel une deuxième dépression hydrodynamique peut être générée au niveau de l'orifice (32) pour immobiliser la cellule (11), la pointe de contact (37) étant disposée de façon que son extrémité supérieure (38) dépasse dans l'orifice (32) et vient en appui contre une membrane cellulaire (39) de la cellule (11) immobilisée.

2. Dispositif selon la revendication 1, **caractérisé en ce que** la pointe de contact (23, 37) est disposée de manière interchangeable.

3. Dispositif selon la revendication 1 ou la revendication 2, **caractérisé en ce que** le canal d'aspiration (33) s'étend dans le substrat (31).

4. Dispositif selon la revendication 1 ou la revendication 2, **caractérisé en ce que** le canal d'aspiration (33) s'étend au moins par segments dans une unité de contact (36) qui contient également la pointe de contact (37).

5. Dispositif selon la revendication 4, **caractérisé en ce que** l'unité de contact présente une partie supérieure (51) interchangeable contenant la pointe de contact (37) ainsi qu'une partie inférieure (52) réutilisable comprenant l'électrode (43) et un premier segment (54) du canal de contact (41).

6. Dispositif selon l'une des revendications 1 à 5, **caractérisé en ce qu'**un espace annulaire (58) affecté au canal d'aspiration (33) est formé autour de la pointe de contact (37).

7. Dispositif selon l'une des revendications 1 à 6, **caractérisé par** une unité de positionnement (21) pour positionner une cellule (11) sur l'orifice (32).

8. Dispositif selon l'une des revendications 1 à 7, **caractérisé en ce que** le substrat (31) est disposé dans un canal d'écoulement (16), de préférence fait partie intégrante d'une paroi du canal d'écoulement (16), dans lequel le liquide (14) est amené selon un écoulement de transport (26) défini et/ou réglable à l'orifice (32).

9. Dispositif selon l'une des revendications 1 à 8, **caractérisé par** une unité de détecteur (18) qui réagit à un paramètre des cellules (11) et qui commande l'unité de positionnement (21) et/ou le canal d'aspiration (33), l'unité de détecteur (18) étant de préférence disposée en amont de l'orifice (32).

10. Dispositif selon la revendication 8 ou la revendication 9, **caractérisé en ce qu'**une unité de microperfusion (19) est disposée en amont de l'orifice (32) afin de distribuer des substances de test à une cellule (11) immobilisée.

11. Procédé de mise en contact électrique de cellules biologiques (11) en suspension dans un liquide (14), comprenant les étapes consistant à :
- immobiliser une cellule (11) au-dessus d'un orifice (32) prévu dans un substrat (31), et
- mettre en contact la cellule (11) immobilisée par le biais d'au moins une électrode (43), l'électrode (43) venant en contact électrique par une pointe de contact (37) avec la cellule (11) et une première dépression hydrodynamique étant appliquée à une membrane cellulaire (39) de la cellule (11) par le biais d'un canal de contact (41) aboutissant à la pointe de contact (37),
**caractérisé en ce que** la cellule (11) est immobilisée en générant une deuxième dépression hydrodynamique dans un canal d'aspiration (33) sur l'orifice (32), les première et deuxième dépressions hydrodynamiques étant commandées séparément.

12. Procédé selon la revendication 11, **caractérisé en ce** la pointe de contact (37, 23) est changée avant chaque mise en contact.

13. Procédé selon l'une des revendications 11 et 12, **caractérisé en ce que** le liquide (14) est amené à l'orifice (32) par un canal d'écoulement (16) avec un écoulement de transport (26) réglable.

14. Procédé selon la revendication 1, **caractérisé en ce que** la zone membranaire ponctuelle de la membrane cellulaire (39) de la cellule (11) immobilisée circonscrite par la pointe de contact (37) est perforée à l'aide de la première dépression hydrodynamique.
